(19)

Europäisches Patentamt
European Patent Office
Office européen des brevets

(11) **EP 4 124 875 A1**

(12) **EUROPEAN PATENT APPLICATION**

(43) Date of publication:
**01.02.2023  Bulletin 2023/05**

(51) International Patent Classification (IPC):
**G01R 33/28** (2006.01)    **A61B 6/03** (2006.01)
**A61N 5/10** (2006.01)    **A61B 6/00** (2006.01)
**A61B 5/024** (2006.01)    **A61B 5/08** (2006.01)

(21) Application number: **21187585.1**

(22) Date of filing: **26.07.2021**

(52) Cooperative Patent Classification (CPC):
**G01R 33/283; A61B 5/02416; A61N 5/1049;**
A61B 5/02427; A61B 5/0816; A61N 2005/1059

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **WEISS, Steffen**
  **5656 AE Eindhoven (NL)**
• **SENEGAS, Julien Thomas**
  **5656 AE Eindhoven (NL)**

(74) Representative: **Philips Intellectual Property &
Standards**
**High Tech Campus 52**
**5656 AG Eindhoven (NL)**

(54) **CAMERA-BASED PATIENT MONITORING IN MEDICAL IMAGING**

(57)    The present invention relates to a medical imaging system (1) comprising a camera (21) for monitoring a subject (14) under examination in an examination zone (11) of the system, and a mirror assembly (22) in the examination zone to reflect light from a target area on the body of the subject onto the camera. The mirror assembly (22) comprises a curved reflective surface and/or a plurality of reflective surfaces oriented in different directions, such that light is reflected from the target area into the camera by magnifying the reflected image and/or by projecting multiple copies of the same target area into different areas of the camera image and/or such that light is both reflected from the target area into the camera as reflected from a light source at a different position as the camera onto the target area.

FIG 1

EP 4 124 875 A1

## Description

FIELD OF THE INVENTION

The present invention relates to the field of medical imaging, e.g. magnetic resonance imaging, computed tomography imaging and the like, and, more specifically, the invention relates to a medical imaging (or: diagnostic imaging) system adapted for camera-based patient observation and/or monitoring of parameters of the patient, such as vital signs and/or movement, based on camera observation and to a related method.

BACKGROUND OF THE INVENTION

Monitoring systems to monitor an examination zone of a medical imaging system, e.g. a magnetic resonance imaging system, with a camera are generally known in the art. Such systems can be used to monitor a patient undergoing examination, e.g. while inside a magnet bore of an MRI system, for various purposes, such as visual patient monitoring by an operator or automated monitoring based on image processing. For example, a camera for imaging the patient during a magnetic resonance imaging examination may be mounted outside the bore of the MRI system, e.g. on or adjacent to a protective cover of the system, e.g. on a flange of the bore to keep the complexity of integration into the MRI system as low as possible and/or to avoid any reduction of the free bore diameter.

Many variations on, and extensions of, this concept are known as well. For example, a mirror may be installed in the bore of the MRI system; such that a camera can observe the patient, e.g. the patient's face, even when no direct line of sight is available or would be difficult to achieve. In many configurations the camera system is mounted relatively far away from the region to be observed, and the optical axis of the camera may be, generally, oriented along, or may have a major component in, the direction of the longitudinal axis of the magnet bore. Since a patient is typically, or at least very often, examined with the longitudinal axis of the body substantially aligned with the longitudinal axis of the bore, the use of such mirror can be particularly advantageous in providing a better view to the camera system.

In addition to allowing an operator to observe the patient, e.g. to detect signs of distress, to ensure that the patient remains awake or to assess adherence to an imaging protocol (for example, in functional imaging of the brain), the camera system may also be used in conjunction with an image analysis system to determine useful parameters, for example to detect and/or quantify movement, breathing, heart rate or other cardiac parameters, and/or other vital signs and/or parameters generally indicative of the patient's state. For example, when motion is detected, e.g. in a generic sense or specifically inferred from a detected respiratory and/or cardiac motion or phase, data that is concomitantly acquired by the MRI system can be labeled to be discarded, annotated and collated as function of the detected (respiratory/cardiac) phase, and/or may be corrected such as to compensate for the detected motion.

Other illustrative applications may include remote photoplethysmography (rPPG), mood detection, video-based talking detection, etc. , ...). Furthermore, it is noted that cardio-triggering is not only useful for cardiac MRI, but may also be applied to various other MRI imaging protocols. A common application in neuro-imaging is to suppress artefacts in head and neck scans caused by pulsatile flow of blood and the cerebrospinal fluid. Another common application is quantitative measurement of blood flow in the carotid artery.

For example, JP H0928689 discloses a mirror installed in the bore of an MRI system and a television camera for taking an image of a subject mirrored on the mirror. A computer analyzes the image and detects the motion of the subject. The computer determines whether the MR data is to be discarded, taken again or collected, e.g. depending on whether the subject moves out of a limited range. Alternatively, MRI parameters can be optimized to cancel the influence of the motion of the subject.

SUMMARY OF THE INVENTION

It is an object of embodiments of the present invention to provide in good and efficient means and methods for monitoring a subject in a medical imaging system (e.g. magnetic resonance imaging system, computed tomography imaging system, positron emission imaging system, single photon emission computed tomography system; without limitation to these examples) using a camera system, and/or for quantifying vital signs, movement and/or other parameters of interest indicative of the subject based on an output of the camera system.

It is an advantage of embodiments of the present invention that better illumination can be achieved of the area of the imaged subject's body that is being observed by the camera system.

It is an advantage of embodiments of the present invention that a high flexibility, e.g. more degrees of freedom, can be achieved in configuring the camera system, e.g. both in terms of illumination of the target area and in terms of field of view of the camera, by allowing an optimization of the optical path from a light source to the target area and/or from the target area to the camera. It therefore also an advantage that the camera system can be easily adapted to different use cases, e.g. imaging protocols, and variation in characteristics of the imaged subject.

It is an advantage of embodiments of the present invention that a good signal indicative of breathing and/or cardiac motion of the subject can be derived, e.g. having an advantageously low signal to noise ratio and/or in a robust and reproducible manner, on the basis of images acquired by camera observation. Such signal may be advantageously used to improve the quality of the imaging procedure, e.g. for respiratory-triggered MRI, motion compensation in the reconstruction process and the like.

It is an advantage of embodiments of the present invention that obstacles in the examination zone (e.g. inside the scanner bore), such as auxiliary coils, parts of the subject's body and/or other equipment used during the imaging process, can be circumvented by a mirror or mirrors to enable a good illumination of a target area on the body and imaging thereof by the camera. Furthermore, it is an advantage that the use of additional mirrors to configure such optical paths can be avoided and/or that the mirrors that are used can be configured without blocking each other's optical path, e.g. while still achieving a good illumination and camera field of view.

It is an advantage of embodiments of the present invention that the subject under examination can view information or entertainment presented on a screen and/or by a projector, in which the camera observation does not block the subject's view or even enables (e.g. at least contributes to) such viewing as an additional feature thereof.

It is an advantage of embodiments of the present invention that an MRI system that is preinstalled, e.g. generally is being used, can be retrofitted easily in accordance with embodiments of the present invention, e.g. by simply replacing a mirror or mirror assembly of a pre-existing camera system (and potentially finetuning already configurable optical and/or software settings).

It is an advantage of embodiments of the present invention that the usable camera detector area, e.g. the available pixels, of the camera system (without limitation thereto, e.g. the same principles apply to analog systems), can be more optimally used by magnifying a part of interest of the subject's body using a mirror or mirrors that provide a magnification.

It is an advantage of embodiments of the present invention that a better view of the targe area to be observed by the camera can be achieved without decreasing the distance of the camera to the target area. For example, it may be preferable to set up the camera (and/or illumination source) outside the examination zone (e.g. outside, or at an edge of, the scanner bore) such as not to interfere with the electromagnetic (radiofrequency) and/or magnetic field properties of the system, or at least to minimize the impact thereon.

It is an advantage of embodiments of the present invention that physiological or other (e.g. related to motion) parameters of a subject can be monitored accurately and robustly during an MRI examination. For example, subtle intensity changes in a camera image or features derived therefrom can be analyzed to extract cardiac, respiratory and/or (more generic) motion signals. For example, for some of such applications, it may be desirable to have a broad view of a part of the subject's body, e.g. the face and/or head, whereas the long distance between camera, when it is set up in a practical and technically less confounding position, e.g. at an end of the magnet bore flange, may typically reduce the viewing angle, e.g. such that the number of usable image pixels for processing may be rather low. Furthermore, it may be difficult or less efficient to achieve good illumination of the monitored body part. These challenges can be overcome, or at least reduced, by embodiments in accordance with the present invention.

A system and method in accordance with embodiments of the present invention achieves the above objective.

In a first aspect, the present invention relates to a medical imaging system, e.g. a magnetic resonance imaging system, computed tomography imaging system, and/or other such diagnostic imaging systems. The system comprises a camera for monitoring a subject when undergoing an examination while positioned in an examination zone of the system by acquiring at least one image. The system comprises a mirror assembly arranged in the examination zone to reflect light from a target area on the body of the subject onto the camera. The mirror assembly comprises a curved reflective surface and/or a plurality of reflective surfaces oriented in different directions. The curved reflective surface and/or the plurality of reflective surfaces are adapted to reflect light from the target area into the camera by magnifying the reflected image and/or by projecting multiple copies of the same target area into different areas of the image acquired by the camera and/or to both reflect light from the target area into the camera as well as reflect light from at least one light source at a different position as the camera onto the target area.

Thus, the curved reflective surface and/or the plurality of reflective surfaces may be adapted to reflect light from the target area into the camera by magnifying the reflected image.

Additionally or alternatively, the curved reflective surface and/or the plurality of reflective surfaces may be adapted to reflect light from the target area into the camera by projecting multiple copies of the same target area into different areas of the image acquired by the camera.

Additionally or alternatively, the curved reflective surface and/or the plurality of reflective surfaces may be adapted to both reflect light from the target area into the camera as well as reflect light from at least one light source at a different position as the camera onto the target area. In other words, one reflective surface may reflect light from a light source onto the target area and from the target area onto the camera, or one reflective surface may reflect light from a light source onto the target area and a different reflective surface may reflect light from the target area onto the camera. Clearly, this does not preclude the use of more than one reflective surface, each one (or a subset thereof) participating in both the inbound and outgoing path with respect to the target, nor does it preclude the use of more than one dedicated reflective surface for illumination and/or more than one dedicated reflective surface for the outgoing optical path from target to camera.

In a medical imaging system in accordance with embodiments of the present invention, the mirror assembly may comprise a first mirror part, in which this first mirror part comprises at least one reflective surface that is adapted to provide a view of the target area to the camera.

In a medical imaging system in accordance with embodiments of the present invention, the first mirror part may comprise a plurality of reflective surfaces, each adapted to reflect the same target area onto the camera, such that the target area is replicated via different optical paths in the acquired image.

In a medical imaging system in accordance with embodiments of the present invention, the first part may comprise a reflective surface forming a convergent mirror to provide a magnified view of the target area to the camera.

In a medical imaging system in accordance with embodiments of the present invention, the convergent mirror may be a spherical or parabolic mirror, or may have a spherical or parabolic profile in at least one direction.

A medical imaging system in accordance with embodiments of the present invention may comprise at least one light source for emitting a light beam into the examination zone, in which the mirror assembly comprises a second mirror part (e.g. distinct from the first mirror part) that comprises at least one reflective surface, which is oriented differently than the reflective surface of the first mirror part; to reflect the light from the light source onto the target area.

In a medical imaging system in accordance with embodiments of the present invention, the second mirror part may comprise a plurality of reflective surfaces with different orientations, such that light from a plurality of light sources is reflected onto the target area by corresponding reflective surfaces of the second mirror part.

In a medical imaging system in accordance with embodiments of the present invention, the mirror assembly may comprise a third mirror part for reflecting an image presented by a screen (which may be part of the imaging system as well) outside the examination zone to the subject such that the subject can visually receive entertainment, instructions and/or information via said screen.

A medical imaging system in accordance with embodiments of the present invention may comprise a radiofrequency transmitter/receiver head-coil assembly for acquiring magnetic resonance signals from the subject's head region, and the mirror assembly may be adapted for attaching to the head-coil assembly or may be integrated therein.

A medical imaging system in accordance with embodiments of the present invention may comprise an arm assembly, e.g. comprising one or more pliable structures and/or joints to provide some freedom in configuration of the orientation and/or position, adapted for attaching to (or attached to) a mount point on a (stationary part of a) gantry or magnet bore enclosure, or on a patient couch or other auxiliary equipment for use in the examination zone of the medical imaging system. Thus, the arm attachment is adapted for mounting in or on (e.g. adapted for attaching to or attached to; e.g. for removably attaching to) the medical imaging system. The mirror assembly may be adapted for attaching to, or may be attached to, the arm assembly, e.g. such that mirror assembly is supported, via the arm assembly, by the mount point. The arm assembly may be a rigid structure, but may also, as already mentioned, be adapted for providing freedom to configure the position and/or orientation of the mirror assembly with respect to the mount point, e.g. via one or more hinges, other types of mechanical joints (having at least a degree of freedom) and/or flexible or pliable structures.

In a medical imaging system, particularly in case of a magnetic resonance imaging system, in accordance with embodiments of the present invention, the camera may be integrated in, or mounted on, a flange end region of a magnet bore housing of the system. The light source or light sources may be integrated in, or mounted on, said flange end region as well.

A medical imaging system in accordance with embodiments of the present invention may comprise an image processor adapted to process image information acquired by the camera to derive information about the subject.

In a medical imaging system in accordance with embodiments of the present invention, the target area may be a part of the forehead or a cheek of the subject or part thereof, and the image processor may be adapted to derive a photoplethysmography signal from images acquired by said camera.

In a second aspect, the present invention relates to a head-coil attachment adapted for attaching to a radiofrequency transmitter/receiver head-coil assembly for acquiring magnetic resonance signals from a subject's head region when examined by a magnetic resonance imaging system. The head-coil attachment comprises a mirror assembly to reflect light from a target area on the body of the subject onto a camera of the magnetic resonance imaging system. The mirror assembly comprises a curved reflective surface and/or a plurality of reflective surfaces oriented in different directions. The curved reflective surface and/or plurality of reflective surfaces are adapted to reflect light from the target area into the camera by magnifying the reflected image and/or by projecting multiple copies of the same target area into different areas of the image acquired by the camera and/or to both reflect light from the target area into the camera as reflect light from at least one light source at a different position as the camera onto the target area.

In a further aspect, the present invention relates to an arm attachment, e.g. comprising one or more pliable structures and/or joints to provide some freedom in configuration of the orientation and/or position, adapted for attaching to a mount point on a (stationary part of a) gantry or a magnet bore enclosure, or on a patient couch or other auxiliary equipment for use in the examination zone of a medical imaging system. Thus, the arm attachment is adapted for mounting in or on (e.g. attaching to; optionally removably attaching to) a medical imaging system for examining a subject when positioned in an examination zone of the system. The arm attachment comprises a mirror assembly to reflect light from a target area on the body of the subject onto a camera of the medical imaging system. The mirror assembly comprises a curved reflective surface and/or a plurality of reflective surfaces oriented in different directions. The curved reflective surface and/or plurality of reflective surfaces are adapted to reflect light from the target area into the camera by magnifying the reflected image and/or by projecting multiple copies of the same target area into different areas of the image acquired by the camera and/or to both reflect light from the target area into the camera as reflect light from at least one light source at a different position as the camera onto the target area.

In a third aspect, the present invention relates to a radiofrequency transmitter/receiver coil assembly for acquiring magnetic resonance signals from a subject's body when examined by a magnetic resonance imaging system. The radiofrequency transmitter/receiver coil assembly comprises a mirror assembly to reflect light from a target area on the body of the subject onto a camera of the magnetic resonance imaging system. The mirror assembly comprises a curved reflective surface and/or a plurality of reflective surfaces oriented in different directions. The curved reflective surface and/or plurality of reflective surfaces are adapted to reflect light from the target area into the camera by magnifying the reflected image and/or by projecting multiple copies of the same target area into different areas of the image acquired by the camera and/or to both reflect light from the target area into the camera as reflect light from at least one light source at a different position as the camera onto the target area.

In a fourth aspect, the present invention relates to a method for monitoring a subject when undergoing a medical imaging examination (e.g. an MRI, CT or other type of medical imaging examination) while positioned in an examination zone of the medical imaging system. The method comprises providing a mirror assembly in the examination zone, illuminating a target area on the body of the subject, and acquiring at least one image by a camera to observe the target area via a reflective surface of a first part of the mirror assembly. Illuminating the target area comprises reflecting light from a light source onto the target area via a reflective surface of a second part of the mirror assembly and/or acquiring the image comprises observing the target area as magnified by a curved reflective surface of the first part of the mirror assembly and/or as replicated in different regions of the acquired image by reflections via a plurality of differently oriented reflective surfaces of the first part of the mirror assembly.

The independent and dependent claims describe specific and preferred features of the invention. Features of the dependent claims can be combined with features of the independent claims and with features of other dependent claims as deemed appropriate, and not necessarily only as explicitly stated in the claims.

BRIEF DESCRIPTION OF THE DRAWINGS

FIG 1 shows a medical imaging system, e.g. a magnetic resonance imaging system, in accordance with embodiments of the present invention.
FIG 2 shows a photograph of a flange section of an MRI scanner bore, into which a camera and light sources are integrated, in accordance with embodiments of the present invention.
FIG 3 shows a camera image, by which a reflection of a patient's forehead can be monitored via a mirror on a head coil of an MRI system.
FIG 4 illustrates light paths from a light source onto a patient's forehead and from the patient's forehead to a camera, by means of mirror, in a mirror arrangement as known in the art.
FIG 5 illustrates light paths from a light source onto a patient's forehead and from the patient's forehead to a camera, in which each of these paths is facilitated by a separate reflective surface, in accordance with embodiments of the present invention.
FIG 6 shows an MRI head coil, having a frontal opening through which the face of a patient (or a part thereof) can be observed, as known in the art.
FIG 7 shows a mirror assembly comprising a plurality of reflective surfaces to replicate a target area of interest on the body of a subject during MRI examination into different regions of an image as acquired by a camera, in accordance with embodiments of the present invention.
FIG 8 shows a further example of a mirror assembly comprising a plurality of reflective surfaces to reflect light from light sources onto a target area of interest on the body of a subject during MRI examination as well as reflect a view of the target area onto a camera, in accordance with embodiments of the present invention.
FIG 9 illustrates a ray geometry of a curved mirror, for use in a system or device in accordance with embodiments of the present invention.
FIG 10 illustrates a method in accordance with embodiments of the present invention. The drawings are schematic and not limiting. Elements in the drawings are not necessarily represented on scale. The present invention is not necessarily limited to the specific embodiments of the present invention as shown in the drawings.

DETAILED DESCRIPTION OF EMBODIMENTS

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

In a first aspect, the present invention relates to a medical imaging system, e.g. a magnetic resonance imaging system, with an examination zone and comprising a camera system for monitoring a subject when undergoing an examination while positioned in the examination zone.

While aspects of the invention will be discussed in detail with respect to a magnetic resonance imaging system, it will be understood that embodiments of the present invention may equally apply to different types of medical imaging system, such as a computed tomography imaging system, a single photon emission computed tomography system, a positron emission tomography system, and/or other medical imaging systems. It will also be understood that the imaging system may be combined with other medical systems, e.g. to deliver a therapy (for example radiotherapy), to support a surgical procedure or to perform another kind of medical intervention. Thus, images of the subject may be acquired by the system to guide a therapy or other medical intervention. Furthermore, where reference is made to typical components of a magnetic resonance imaging system, such as a magnet bore enclosure, it will be understood that other embodiments may relate to different imaging modalities, in which such components can be substituted by analogous components, e.g. having a similar or the same function, or generally a similar or the same form. For example, where reference is made to a magnet bore enclosure, it will be understood that this may be considered to be analogous to a gantry of a computed tomography imaging system, in so far the analogy would make sense. Likewise, where reference is made to a head-coil assembly, it will be understood that this may be analogous to a head enclosure for use in other types of systems (or even a different type of head enclosure that can be used in MRI as well), such as a stereotactic head frame for use in planning and executing a radiotherapy or surgical procedure, a radiotherapy mask, or the like. These remarks apply equally to further aspects of the invention discussed further hereinbelow, e.g. to a method in accordance with embodiments.

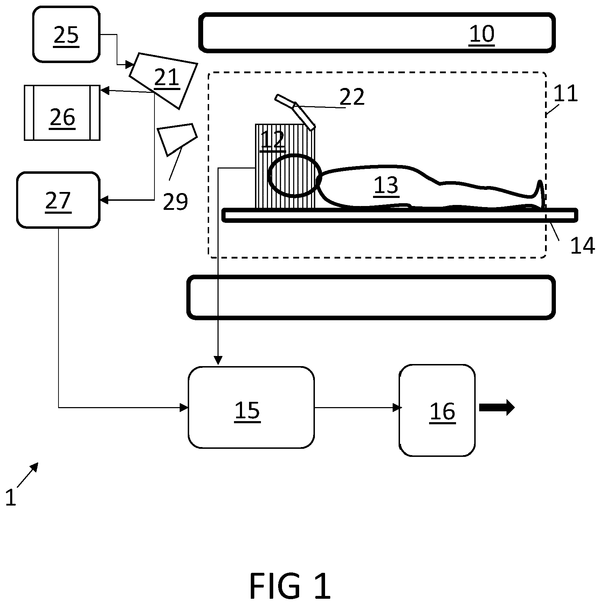

FIG 1 schematically shows a magnetic resonance imaging system 1 in accordance with embodiments of the present invention. The magnetic resonance examination system comprises a primary magnet assembly 10, which defines the examination zone 11, e.g. the examination zone may be formed by a volume where the magnetic field conditions, as substantially created and controlled by the magnet assembly, are suitable for magnetic resonance imaging. The examination zone may thus correspond to (at least a usable portion of) the volume enclosed by a magnet bore of the system (without limitation, e.g. principles of the present invention equally apply to open bore systems and other, less frequently used, magnet assembly configurations).

A subject, e.g. a patient, to be examined 13 may, in use of the system, be positioned on a patient couch 14 in the examination zone. The primary magnet assembly may comprise magnet windings, e.g. coaxial (e.g. superconductive) windings, to generate a stationary uniform magnetic field in the examination zone. The examination zone may be a cylindrical volume encompassed by these magnet windings.

The system may comprise a reconstructor 15 to reconstruct magnetic resonance image(s), e.g. tomographic MRI images, from magnetic resonance signals acquired by the system in use. The reconstructed images may be provided via an output 16 for viewing, processing or storage.

Auxiliary equipment, such as an RF T/R head coil 12 may, in use, be place in the examination zone to acquire magnetic resonance signals from the subject's head. Other auxiliary coil configurations may be used to acquire signals from other body parts or for different use cases, while, typically, signals may also be received by receiver coils already integrated in the housing of the primary magnet assembly.

It will be understood that, in other embodiments, the system may be a medical imaging system for a different imaging modality, e.g. a computed tomography system (without limitation thereto).

The system comprises a camera system that comprises a camera 21. The camera system is adapted to obtain information from the subject being examined, e.g. to obtain vital signs, motion, indicators of distress and the like. Obtaining this information may refer to merely obtaining images to be presented to an operator for evaluation, i.e. to derive the information by observing the images, and/or may refer to acquiring the image for processing, e.g. by an electronic processing device, to determine a useful value or indicator that represents a parameter or parameters of interest.

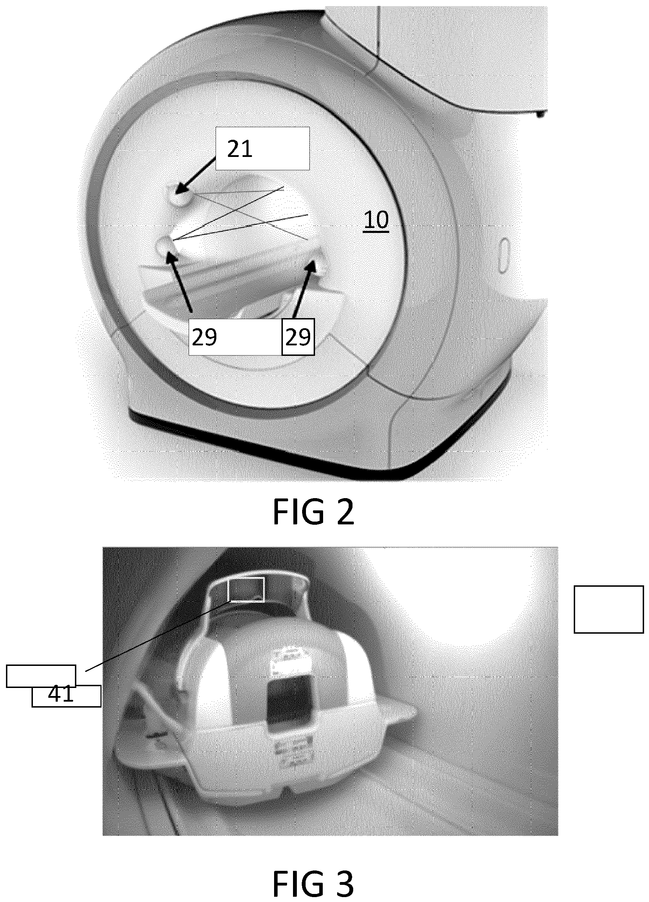

The camera 21 may be mounted close to one entry of the examination zone. For example, the camera may be integrated in, or mounted on, a flange of the MR bore (e.g. such that the usable free bore diameter is not affected or only minimally reduced, and/or to avoid or minimize interference with the operation of the MR system). This is for example illustrated by the photograph in FIG 2, which also shows the integration of (optional) illuminating lights 29 in this flange. The camera system may also comprise a camera control 25 to control the camera 21, e.g. to adjust parameters such as orientation of the optical axis, focal length, etc . The camera system may comprise a display 26 to display images of the inside of the examination zone 11 acquired by the camera 21 (raw, or after suitable processing). This enables an operator to visually monitor the subject in the examination zone.

The images acquired by the camera 21 may be provided to an image processor 27 (which may for example be implemented in software) to derive information about the subject from the acquired camera image or images.

The image processor 27 may be adapted to process image information acquired by the camera system, e.g. to perform static or dynamic image analysis to obtain information from the patient, such as vital signs of the patient, and/or motion of the patient, and/or signs of distress of the patient (or, more generally, patient mood detection), and/or photoplethysmography (PPG), and/or video-based detection of talking (or recognition of speech, e.g. of simple words or instructions based on facial features). Information on motion of the patient may include, for example, respiratory motion and/or cardiac motion, e.g. indicating the phase of respiratory and/or cardiac cycle phase. For example information on motion of the patient may be derived from image information of the outer hull of the patient's body. The information may be determined by processing (e.g. by an image-based motion detector) and/or by (direct) visual monitoring of the patient via the system by an operator or staff member.

Respiratory and/or cardiac phase information (and/or more generic information indicative of motion) may be provided to the reconstructor 15 to correct the acquired magnetic resonance signals for motion and/or apply motion corrections to the reconstructed magnetic resonance images. For example, a cardiac trigger signal may be determined based on video signals from the camera. Cardiac triggering is particularly useful for cardiac MRI, for obvious reasons, but may also be applied more generally. For example, in neuro-imaging, artefacts in scans of the head and/or neck caused by pulsatile flow of blood and/or cerebrospinal fluid may be suppressed or reduced by such triggering technique or other compensation approach based on a cardiac phase signal. This may also be useful for quantitative measurement of blood flow in the carotid artery. Furthermore, a PPG signal can be extracted from the video signal by analyzing subtle intensity changes of skin pixels, e.g. in the face of a subject, such as at the forehead or at the cheeks.

In a magnetic resonance imaging system in accordance with embodiments of the present invention, the camera system may also comprise one or more light sources 29. While embodiments that rely on passive lighting for imaging are not necessarily excluded, it will be understood by the skilled person that lighting conditions can be better controlled, and imaging can be more effective, when using active lighting.

The light source 29 may be configured and positioned for directing its light beam directly into/onto the examination zone, possibly being supported by the mirror(s) or reflective surface(s). The use of multiple mirrors (or a faceted mirror) or a mirror that is curved may furthermore allow different areas on the subject to be illuminated effectively with a single light source and/or allow both illuminating light from a light source or sources to be reflected onto the target area of the subject and observation thereof (via reflected image) by the camera, even if the camera and light(s) are not at a same location or in very close proximity to each other.

The light source and/or the camera may be located outside the examination zone, or on or near an edge region thereof. This can simplify the configuration of the magnetic resonance imaging system (e.g. avoiding or reducing interference with the RF and magnetic field operation of the system) and may provide for more free bore width in the examination zone. For example, for a cylindrical bore system, both camera and light source (or either one thereof individually) may be located at the flange of the bore enclosure at one end of the bore, which may leave the other end substantially free, e.g. to allow unimpeded access to the examination zone (for bringing the patient and/or auxiliary equipment into the examination zone), and reducing a potential claustrophobic effect on the subject, and thus possible discomfort, while being imaged by the system.

The light source(s) 29 may be located next to, or in the vicinity of, the camera, e.g. adjacent to each other at a flange end of the bore or separated by an angle but generally in the same flange region in the longitudinal direction, e.g. separated by an angle around the longitudinal axis, see e.g. FIG 2. If the light source and camera are positioned relatively close together, a mirror or reflective surface may be used to bring the emitted light from the source to the examination zone (e.g. a body part to be observed) and back again from the observed examination zone to the camera, e.g. such that the same mirror is used effectively twice in the optical path, thus reducing the number of required mirrors (however, without limitation thereto). Positioning the light source and camera in close proximity to each other may have further advantages, such as minimizing the impact on (reduction of) available bore width and optionally sharing cables or conduits for power supply and/or control signals (which may also simplify design of the system in terms of avoiding or reducing RF and/or magnetic field interference). However, it is an advantage of embodiments that a good illumination can be achieved as well as a good view by the camera with few or only a single mirror even if the camera and the light source(s) are separated by a substantial distance, e.g. a different angular positions around the longitudinal axis (e.g. at least 10° separation, e.g. at least 20° separation, or even substantially more, e.g. in the range of 30° to 120°, e.g. in the range of 45° to 90°, e.g. in the range of 30° to 60°).

In a magnetic resonance imaging system in accordance with embodiments of the present invention, the camera (or cameras) may be adapted to operate (e.g. substantially exclusively sensitive to) light in a (e.g. narrow) infrared wavelength range and outside the visible wavelength range.

In a magnetic resonance imaging system in accordance with embodiments of the present invention, the camera (or cameras) may be adapted to operate (e.g. substantially exclusively sensitive to) in the visible wavelength range, e.g. sensitive to a broad white light spectrum, or part thereof, e.g. a color band. The camera may be adapted to acquire monochrome information, or may be a color camera, e.g. adapted to detect, preferably independently and substantially simultaneously, different color components, e.g. a red, green and blue component (without limitation thereto). The camera may also be adapted to detect a relatively large (e.g. more than three) spectral components, e.g. may be a multispectral camera.

The light source(s) may emit light in a spectrum suitable for the camera, e.g. a broadband white light may provide illumination for a monochrome or color camera in the visible range to operate. Likewise, an infrared light source may be used to emit infrared light in a spectral range in which the infrared camera is sensitive. It will be understood that the spectra of the light source and the camera are not necessarily identical or not even necessarily closely related, e.g. the spectrum of the light source may be broader in so far that sufficient overlap exists with the spectrum to which the camera is sensitive.

The camera may be an analog camera or, preferably, a digital camera, e.g. comprising an array of pixel light detectors.

The system comprises a mirror assembly 22 arranged in the examination zone, to reflect light from a body part of the subject, such as the face or part thereof (e.g. the eyes or an eyeball, a region on the forehead, ...), onto the camera, and/or to reflect light from the light source(s) onto said body part. The mirror assembly 22 comprises one or more mirrors and/or reflective surfaces. The mirror assembly may comprise a (e.g. non-metallic) mirror. The use of the term 'mirror' should not be construed narrowly, e.g. the mirror assembly may be constructed from components that act as a mirror and/or reflector, i.e. reflect light in the spectral range of interest. The mirror assembly comprises at least a part that is adapted to reflect an image from the body part of interest toward the camera (not necessarily excluding additional mirrors along its path toward the camera), i.e. that is suitable for reflecting light without substantial scattering and/or diffusing to be able to form an image of reasonable quality. The mirror assembly may comprise another part or parts that reflect light from a light source(s) onto the body part of interest, but the requirements for this second part or parts may be more relaxed, e.g. scattering or diffusion of the reflected light may be tolerable.

The mirror assembly 22 may be mounted to the inner wall of the examination zone, e.g. of the magnet bore enclosure, and/or on auxiliary equipment, such as the patient table (or: patient couch) or a head coil assembly. For example, the mirror assembly may be attached via an arm assembly to the patient table. Thus, the mirror assembly may be mounted on, or formed as part of, a head T/R coil, e.g. as used for cervical, cranial and/or neuroradiological MR examinations. It is to be noted that integrating the mirror in or on the head coil may avoid costly or complex modification of existing equipment, e.g. of the scanner bore. While a relatively far distance between the camera, e.g. mounted on a flange of the bore, may result in a very limited field of view, e.g. only showing the forehead or part thereof, this may be sufficient for some applications, e.g. to monitor blood pulsation by slight variations in pixel intensity.

A medical imaging system in accordance with embodiments of the present invention may comprise an arm assembly, adapted for attaching to (or that is attached to) a mount point on a (stationary part of a) gantry or magnet bore enclosure, and/or or on a patient couch and/or other auxiliary equipment for use in the examination zone of the medical imaging system. Thus, the arm attachment is adapted for mounting in or on (e.g. adapted for attaching to or attached to; e.g. for removably attaching to) the medical imaging system, or a part thereof (e.g. the patient couch). The mirror assembly may be adapted for attaching to, or may be attached to, the arm assembly, e.g. such that mirror assembly is supported, via the arm assembly, by the mount point. The arm assembly may be a rigid structure, but may also be adapted for providing freedom to configure the position and/or orientation of the mirror assembly with respect to the mount point, e.g. via one or more hinges, other types of mechanical joints (having at least one degree of freedom) and/or a flexible or pliable structure.

While embodiments are described further hereinbelow that relate to a mirror assembly that is integrated in or attached (or attachable) to a head-coil, it will be understood that the same principles apply to different designs of auxiliary equipment, and that similar results can also be obtained when the mirror assembly is mounted on a (inner) wall of the examination zone. For example, instead of supporting the mirror assembly by a head-coil, it can equally be supported by the bore wall above the head-coil (or the head, if no head-coil is used; or above another body part of interest to be observed by camera).

The use of (a) mirror/reflector(s) may be particularly advantageous when the region of interest for observation is blocked from direct view by the camera. For example, an obstacle may be formed by an opaque object, e.g. auxiliary equipment such as local radiofrequency (RF) transmit or receive (T/R) antennas, located in the camera's direct line of sight into the examination zone. Other possible obstacles include parts of the subject's body.

Preferably, the mirror assembly 22 does not interfere with the radio frequency operation of the magnetic resonance imaging system and/or does not perturb the magnetic fields and the RF dynamic transmission field of the MRI system. Non-metallic mirrors may be particularly suitable for achieving this advantage. For example, the non-metallic mirror may be a dielectric mirror, e.g. comprising a stack of layers of different refractive index, e.g. such that a dielectric resonator is formed by the stack. While such arrangement is particularly suitable for a narrow wavelength range, it will be understood that varying the thickness of the layers in the stack can accommodate for reflection of different wavelengths, such that a broadband reflection (or approximation thereof) or reflection of multiple color components can also be easily achieved.

In a magnetic resonance imaging system in accordance with embodiments of the present invention, the mirror assembly may be particularly adapted to reflect light such as to allow the formation of an image of the body part of interest (for camera observation) on the imaging plane of the camera and/or to reflect light from the light source to the body part of interest. Optionally, the mirror assembly, or at least part thereof, may also be transparent or translucent for light in the (human) visual range, i.e. when a different spectral range is used for illumination and camera imaging, such as infrared.

The mirror assembly 22 may thus arrange an optical pathway between a portion of the examination zone, i.e. where the body part is located when the subject is undergoing examination, and the camera, such that the camera can obtain image information from that portion. The mirror assembly may be positioned in the examination zone, e.g. by mounting to the inner wall of the magnet bore enclosure. Alternatively, the mirror assembly may be disposed on auxiliary equipment in the examination zone, such as on a local radio frequency (RF) coil, e.g. when positioned on a patient couch in the examination zone. For example, it may be practical to mount the mirror assembly onto an RF head coil.

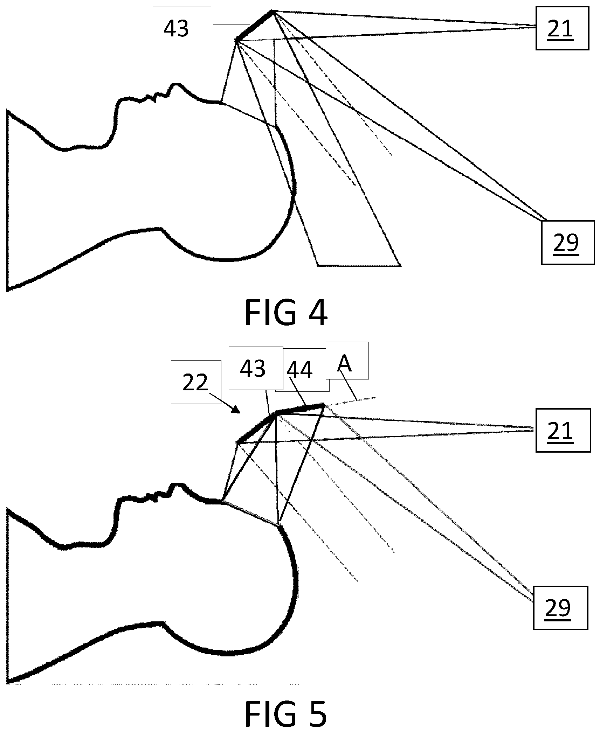

For example, neuro-scans are usually performed with the head of the patient placed inside a head-coil located at the iso-center of the magnet. Neuro coils substantially fully enclose the head and neck of the patient so that a direct view by the camera of the head is mostly obscured. A camera can be mounted at the flange of the bore, e.g. as shown in FIG 2. Its view onto a patient located in the head-coil is depicted in FIG 3. A small part of the forehead of the patient can be observed by a mirror mounted on top of the head-coil, e.g. indicated by the area 41. The substantial enclosure of the head by the head-coil poses the problems by only allowing a limited view, e.g. a small part of the forehead in this example, and makes it more difficult to illuminate the target area properly. For example, it may be difficult to illuminate the forehead directly by the bore lights (see e.g. FIG 2), such that only indirect light scattered by the bore wall reaches the area of interest. The fact that camera and bore lights are located at different positions on the flange of the bore can also contribute to the problem of insufficient illumination. A single flat mirror 43 cannot effectively (or sufficiently) reflect light from the light source 29 onto the target area (e.g. forehead) as well as light from the target area onto the camera 21, as illustrated in FIG 4. In other words, to obtain a good view of the target area by the camera, the mirror cannot effectively or efficiently project light from the bore lights onto the same region.

The mirror assembly 22, in embodiments in accordance with the present invention, is therefore curved or faceted. A faceted mirror assembly may comprise a plurality of reflective surfaces that are not parallel to each other, such that differently oriented parts can reflect light from different volumes in space onto substantially the same target region or vice versa. Thus, light from a light source can be reflected onto the target area and the target area can be observed by a camera at a different position, and/or light from different light sources can be directed to the target area. Likewise, different cameras can view the same area in a multi-camera configuration using differently oriented mirror facets, or a same target area can be replicated in the camera image acquired by the camera to increase the number of available pixels corresponding to the target area, e.g. for algorithmic processing purposes.

For example, FIG 5 schematically shows a configuration in which a faceted mirror is used. The mirror assembly 22 comprises a first mirror 43 and at least a second mirror 44, or multiple additional mirrors. In other words, one or more additional mirrors or mirror segments 44 be added to the first mirror 43 shown in FIG 4, to form such faceted mirror, in which this second mirror(s) has a different tilt angle. The second mirror(s) may be tilted such that it reflects light from the light source to the region of the forehead that is seen by the camera via the first mirror.

In addition to a tilt around a horizontal axis (perpendicular to the longitudinal axis of the scanner system, i.e. around a lateral axis), as indicated by the plane A of the second mirror being not in line with the plane of the first mirror, this second mirror may also be tilted around a vertical axis (in use, the frontal axis when a patient is imaged in a conventional supine position). This is obviously advantageous in an embodiment where the camera and light source are positioned at different angular positions in a same flange region, see e.g. FIG 2, in order to project light onto the target region and image the target region by the camera. The second mirror facet may comprise a plurality of different mirror facets, which differ in angulation (tilt), e.g. to project light from different sources onto the target region. In the example of FIG 2, the light sources are provided at substantially the same vertical position (height), e.g. where the bore is widest in the horizontal plane (without limitation), at the same longitudinal position (the flange end of the bore), but at different sides with respect to the center. Thus, for this example, two second mirrors 44 may have a same angle with respect to the vertical, but would be tilted differently around this vertical axis (e.g. symmetrically with respect to a vertical plane through the iso-center), e.g. each having different tilts around the axis A shown.

Thus the mirror assembly in accordance with embodiments may comprise at least two facet parts, which may be separate mirrors or reflective surfaces or may form part of a single connected mirror and/or reflector structure in which one or more facet edge lines connect the facets (e.g. may be integrally formed as a single mirror). As can be seen from the example above, the mirror or reflective surface may comprise at least three faceted parts, e.g. in the range of 3 to 10, e.g. 3 to 5, e.g. 3 or 4 faceted parts, e.g. at least a reflective source for each camera and each light source (and possible additional reflective surfaces for reflecting multiple copies of the target area onto different areas in the image acquired by a camera, as discussed in detail further below).

The size and shape of the (or each) secondary mirror (or mirror facet) 44 may be adapted to produce an illuminated area that corresponds to (or strongly overlaps with) the region of interest seen by the camera when illuminated by the (or a corresponding) light source. The secondary mirrors may be rectangular, however this is not strictly necessary, e.g. may deviate from a rectangular shape for this purpose. Thus, the size and shape can be determined on the basis of the envisioned position of the mirror assembly in use of the device, the size and shape of the region to be observed, and the position of the light source from which light is received to reflect onto the target region.

It is also noted that the use of further secondary mirror facets (e.g. such that the mirror assembly comprises more than two differently oriented facets) for receiving light from more than one light source can also be advantageous to avoid shadows in the region of interest, e.g. such that each region where a shadow is cast when lighted by one light source is sufficiently illuminated by another.

The first mirror (facet) 43 may be larger than (e.g. each) second mirror 44. Thus, the first mirror may use a large area to be able to project a good image onto the camera, whereas less space is reserved for the second mirror(s) that have an auxiliary function in providing good illumination.

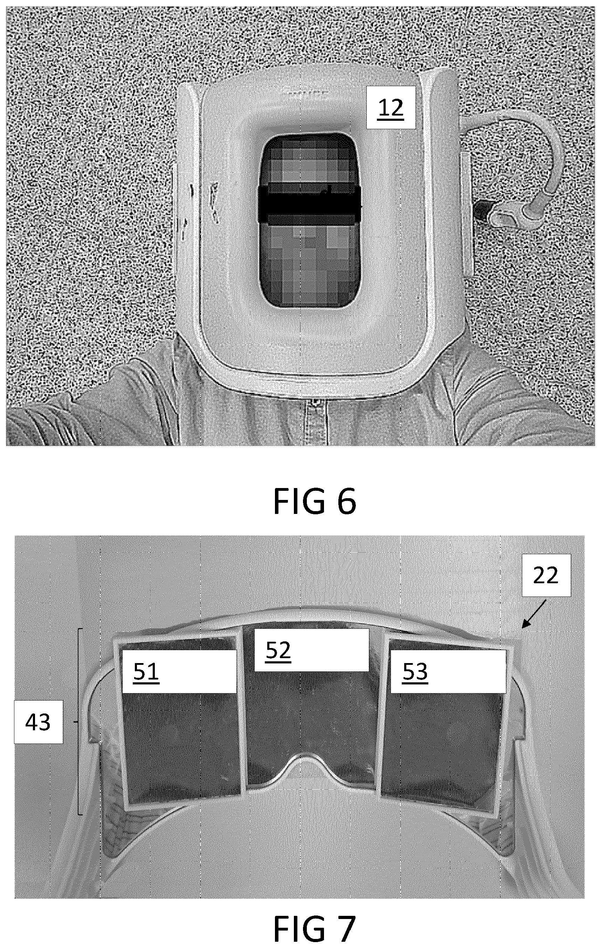

For example, the first mirror 43 may be (laterally) limited in size to a the size of an opening of the head-coil onto which the mirror assembly may be mounted in accordance with embodiments. FIG 6 illustrates a typical head coil assembly 12, in which a frontal opening provides a maximum usable width d for the mirror assembly, e.g. for the first mirror 43. By adapting the width of the first mirror to this width d of the opening, or a close approximation thereof, a good view of the (e.g. full) forehead can be achieved by the camera. This does not necessarily imply that the mirror is strictly limited to this width, e.g. optics and design considerations can motivate a (e.g. slightly) wider design. If the first mirror is curved, e.g. forms a convergent mirror, it may be possible to increase the (usable) width of the mirror even further.

However, since such opening is generally quite heigh in comparison to its width, additional space above or below (in a horizontal plane) the primary mirror 43 can be used for the secondary mirror(s) 44. Furthermore, the space to either lateral side (again in the horizontal plane) can also be used, additionally or alternatively, since a different angulation of the second mirror(s) may allow light to directed to the area of interest from a more acute angle. It is also noted that the use of more than one light source and more than one secondary mirror 44 may be even more advantageous if the light is cast onto the target area in more acute angles, since larger shadows can be cast in such case and compensated by additional illumination paths.

Thus, good illumination of a region to be observed by the camera can be achieved by embodiments, which also enables more robust and/or accurate signal detection based on camera image analysis, e.g. inferring a remote PPG signal from the camera images.

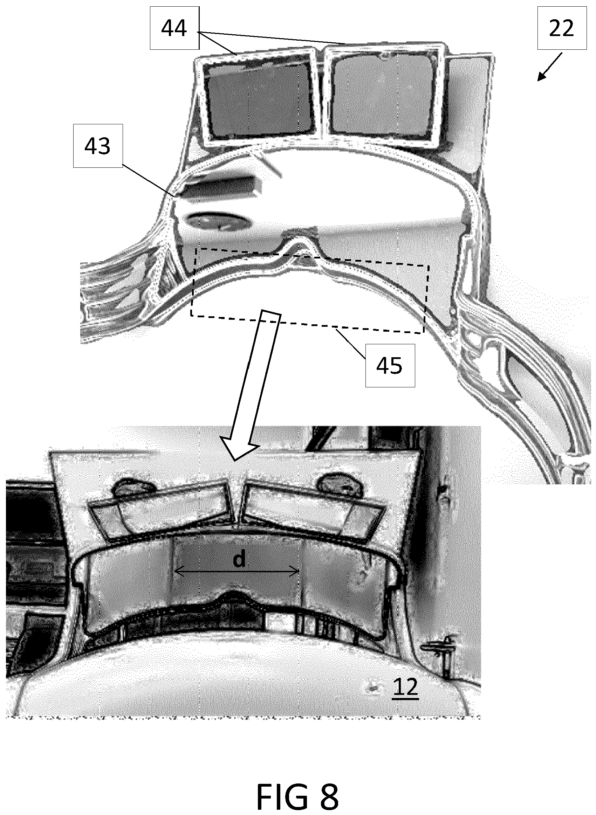

FIG 8 illustrates an example of a mirror assembly 22 for attachment to a head-coil 12 for MRI imaging, as well as a camera image, e.g. as can be detected by a bore camera in use. The mirror assembly comprises a first mirror 43 for reflecting an image of a target area onto the camera, while two second mirrors 44 project light from respective light sources onto the target area.

The mirror assembly 22 comprises one or more mirrors and/or reflective surfaces, e.g. the first mirror or mirror part 43 for reflecting light from a body part of the subject onto the camera. As discussed hereinabove, the mirror assembly 22 may also comprise a second mirror or mirror part 44 for reflecting light from the light source(s) onto said body part. The mirror assembly 22 may also comprise a third mirror or mirror part 45 (conceptually indicated in generally an area below the first part in FIG 8) for reflecting an image presented by a screen to the subject (e.g. onto an eye or eyes of the subject), e.g. for providing entertainment, instructions and/or information to the subject. For example, in use, the mirror 43 in the example of FIG 8 may be positioned generally above the forehead to observe this area by the camera, whereas the secondary mirrors 44 may be positioned higher (in the sense of the cranial direction in use) to illuminate that area. The third mirror part 45 may be added below the first mirror (e.g. in the caudal direction in use), e.g. in use located above the eyes of the subject, to enable the subject to watch a screen (e.g. a display or projection screen). The tilt angle of this third mirror (or mirror facet) may be adapted for this purpose.

It is an advantage of using separate mirror elements (as opposed to an integrally formed mirror assembly comprising multiple, differently tilted facets) that each mirror element may be configured to be adjustable in position and/or orientation, e.g. using a sliding or swivel base. This can provide additional flexibility to adjust the mirror assembly to different use cases, e.g. different positions of the mirror assembly along the longitudinal scanner axis when the camera and light sources are at a fixed position. Nonetheless, an integrally formed construct may offer other advantages, e.g. in terms of robustness and ease of use.

Furthermore, the first mirror part for mirroring the target of interest onto the camera may comprise a plurality of separate parts, e.g. facets or individual mirrors, each adapted to reflect substantially a same area of interest on the subject's body onto the camera. Thus, a region of interest can be replicated via different optical paths in the acquired camera image.

For example, when the target region on the body is relatively small, e.g. the forehead or a part thereof (e.g. a central frontal area of the forehead), it may not be necessary to make an individual mirror facet to image this region very wide. Other examples of regions that could be of interest may include an eyeball, e.g. for eye tracking or alertness monitoring. Thus, a single flat mirror to observe such region may not need to be wider than the opening of the MR head-coil. This leaves space to add additional mirrors or mirror segments, e.g. flat mirror segments that are at slightly different angles with respect to each other. FIG 7 illustrates an example, in which the mirror assembly 22, e.g. the first part 43 thereof, comprises three (without limitation to this number) mirrors 51, 52, 53. The mirror assembly 22 thus may comprise a first mirror part 43 for reflecting light from a body part of the subject onto the camera, which consists of a plurality of mirrors 51,52,53 and/or mirror facets that are adapted, e.g. oriented, to reflect substantially the same target area of a the body part of interest onto the camera from slightly different angles. These mirrors 51,52,53 of the first part may be flat or curved, e.g. having a convergent or focusing shape (or the equivalent thereof achieved by e.g. a grating design). The (e.g. flat) mirrors may be oriented at different angles, such that the same (e.g. substantially overlapping) area is projected onto the camera. Effectively, the camera can thus observe multiple reflections of the same area, e.g. the forehead or part thereof, for example side-by-side in the camera image. This image may then be processed to extract useful information, such as a cardiac signal, which may advantageously improve a signal to noise ratio because the sensor noise of pixels in the camera is typically uncorrelated or at least without strong correlation. This can be used in an embodiment in a standalone manner, or in combination with the second and/or third mirror parts discussed hereinabove. For example, in the example shown in FIG 7, the total width may roughly correspond to the width of an opening in the head coil onto which the mirror assembly can be mounted, while each individual mirror is about the size (and/or shape) of the region of interest, e.g. a frontal and central region of the forehead (without limitation).

For example, this may correspond to the area 41 indicated in the situational overview of FIG 3.

Additionally or alternatively, the mirror assembly 22 or a part comprised therein may be curved, e.g. to form a convergent (focusing) mirror, e.g. a zoom mirror. For example, a relatively far distance between camera and the region of the patient to be observed, e.g. the forehead (for example for enabling remote PPG measurements), for example when the camera is mounted on or near the flange of the magnet bore, can result in a relatively low viewing angle and the region of interest only covering a small amount of camera pixels in the acquired image. This would limit the signal to noise ratio (SNR) for video-based extraction of a signal of interest, such as a cardiac signal.

While this could be overcome at least to some extent by selecting a camera lens with a larger focal length, this would have the disadvantage of limiting the global view of the camera, which can be useful for other applications, such as motion tracking and general patient monitoring.

It is noted that this concept can also be combined with the feature discussed hereinabove in which multiple mirrors or mirror segments are configured to provide a view to the camera of the same area of interest, e.g. substantially overlapping on the same body part or region of interest thereof. In other words, each of the mirror segments may be curved to provide a zoomed reflection.

The images acquired by the camera 21 may be provided to an image processor 27 (which may for example be implemented in software) to derive information about the subject from the acquired camera image or images.

The image processor 27 may be adapted to process the image information acquired by the camera system. For example, the magnification and/or image replication (e.g. of a same target area on the patient into multiple image regions) may provide a plurality of pixels in the image (particularly, more than would be available when no replication and/or optical magnification would be applied) which are suitable for signal extraction of a signal of interest (not necessarily excluding on signal processing that rely on larger-scale image features, e.g. edge detection and the like, which may equally be replicated in the image when using multiple reflections or may be enhanced by magnification). Thus, the signal extraction may be applied to each pixel (or pixel region, or image feature that is replicated in the image), and the results thereof may be combined, e.g. by averaging (without limitation thereto), to produce a more robust and/or more accurate estimate of the signal of interest. It will also be evident that such combination or aggregation may be applied at different stages in the processing algorithm, e.g. pixels (or other image features) may be averaged (or otherwise summarized and/or combined by a suitable measure) to obtain a less noisy representative value, which can be used for subsequent processing to determine the signal of interest, or an intermediate result derived from the image may be combined (over different areas representative of the same image feature, and/or over different intermediates obtained from different pixels, etc., without limitation thereto) before continuing the processing algorithm to arrive at the signal of interest.

Furthermore, it is also noted that the reflective surfaces of the second part 44 for illuminating the area of interest may be curved, e.g. such as to spread out the light received from a light source over the target area. While the mirror of the first part for providing a view to the target area is preferably of a convergent shape (at least if non-flat), a curved mirror of the second part for illumination purposes may have a convergent shape or divergent shape, depending on the optical characteristics of the system. For example, when the distance to the light source is relatively small and/or the light source emits a tight beam, e.g. a laser beam, it may be advantageous to provide a divergent mirror to spread this narrow beam out over the entire target region, whereas for a light beam emitting a broad beam (e.g. a focal source) and at a larger distance, it may be advantageous to provide a convergent mirror to capture more incident light and focus this incident light onto the target area.

It is noted that 'curved', 'convergent', 'divergent' and the like should be interpreted in a functional sense throughout this specification. For example, the skilled person is aware that properties of a curved mirror can be achieved by a flat diffraction grating or similar designs using diffraction and/or structured reflection optics. In other words, a (mechanically) flat diffraction grating or similar optical structure may be considered 'curved' in the optical sense or, at least, entirely equivalent thereto.

By providing a curved mirror or multiple curved mirrors, e.g. convergent mirrors, e.g. parabolic mirrors, and/or by replicating a same area of interest on the body in the camera image by multiple mirrors or mirror facets, generally more pixels become available for monitoring and image processing, e.g. increasing a SNR of a value derived from the image by processing, e.g. a cardiac signal. This also has the advantage that it is not necessary to change hardware of the camera or magnetic resonance imaging system (except, obviously, the mirror assembly itself when considered part of the system). Replication can be achieved by several (e.g. slightly) angulated mirrors, and enlargement by use of a curved mirror.

For example, the mirror assembly may comprise one or more curved mirrors or mirror facets. The curved mirror (or curved region of a mirror, e.g. in embodiments where more functional mirror parts are integrated in a single integrally formed mirror) may be a concave mirror, e.g. a parabolic or spherical mirror. A spherical mirror may have the advantage that it can be more easy and cost-efficient to manufacture, even though embodiments are not limited thereto.

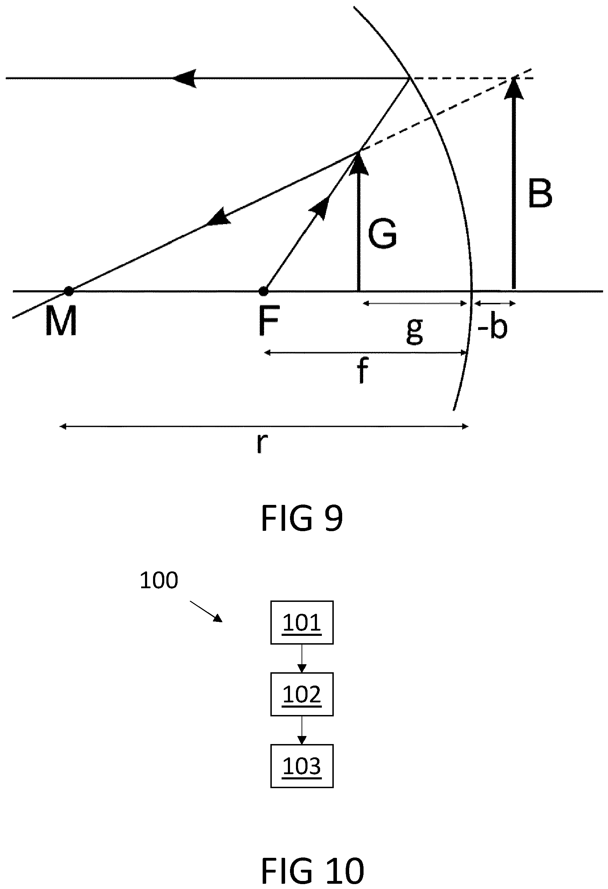

For example, without limitation thereto, this curved mirror or curved mirror part may have a fixed curvature radius $r_H$ at least in a first direction, e.g. the lateral direction (relative to the subject when positioned prone in the imaging procedure, e.g. the horizontal direction perpendicular to the longitudinal axis of the MRI system). The second curvature radius $r_V$ (e.g. perpendicular to the first direction), e.g. in anterior-posterior direction in use, e.g. the vertical direction, may be identical to $r_H$ (thus forming a spherical mirror) or it may differ. The radii are chosen such that, by this mirror or mirror part, a single but enlarged image of the target region, e.g. the forehead or part thereof, is reflected into the camera. The ray geometry for such illustrative zoom mirror is shown in FIG 9. The forehead G of the patient is positioned within the focal length $f=r/2$ of the mirror. This reflects an enlarged virtual image $B$ of the forehead into the camera. The Gaussian lens equation is $\frac{1}{f} = \frac{1}{g} + \frac{1}{b} = \frac{2}{r} \Rightarrow b = \frac{fg}{g-f}$, with $b$ being a negative value because of the virtual image behind the mirror. The magnification $m$ is then given by $m \equiv \frac{B}{G} = -\frac{b}{g} = \frac{f}{f-g}$.

For a given distance $g$ between forehead and mirror, and a desired magnification factor $m$, the corresponding focal length $f$ of the mirror is then given by

$$f = \frac{mg}{m-1}.$$

As an illustrative example, for an embodiment in which the mirror is positioned over the forehead on a head-coil, a distance g of 20 cm may be assumed, such that a desired magnification of e.g. $m=5$ results in a focal length of $f=25$cm and a curvature radius of $r=50$cm. The desired magnification can be varied by selecting another value of r.

Even though both the illustrative embodiment of image replication by multiple mirrors and the illustrative embodiment of image magnification by a zoom mirror may have the advantage of increasing the signal to noise ratio of detection of a property of interest by image processing, e.g. both leading to more pixels in the image collecting light that is relevant for the processing, magnification by a mirror has the additional advantage that more detail in the area of interest, e.g. on the forehead of the patient, can be resolved by the camera. This may be valuable for particular image processing applications, such as motion detection and quantification for use in motion compensation techniques (in reconstructing and/or processing the acquired MRI images). For example, motion detection can be based on pattern matching, which could be more accurate and/or robust if more detail is available in the image. Motion compensation may also be particularly useful as an element of a processing algorithm that is adapted to determine another useful parameter from the observed camera images, e.g. motion compensation of the acquired camera data may improve the reliability and accuracy of cardiac signal detection based on said camera images.

Regarding the mirrors and/or mirror facets of the mirror assembly discussed hereinabove, it is noted that a plurality of such differently directed and/or oriented (and/or distorting by magnification) reflective surfaces, placed, in accordance with at least some embodiments, in or near the field of view of the subject undergoing examination, may have a confusing or disturbing impact on the subject's experience. For example, the subject may see multiple replicated views or a distorted view of his environment through such mirrors. Frequently, a patient undergoing examination may watch video content on a screen, or may be instructed to pay attention to information and/or instructions on the screen, which is visible to the subject via a mirror in front of the eyes. In such circumstances, it will be understood that additional mirrors and/or curved mirrors in the eyesight of the subject can be particularly distracting or even discomforting.

The use of dielectric mirrors may overcome this problem, e.g. in combination with camera imaging outside the visible spectrum or in only a narrow band (e.g. reducing the confusing visual information reflected to the patient to a single color that can be more easily ignored). For example, a dielectric mirror can be designed to be only reflective to a specific narrow wavelength band, e.g. in the infrared range or a narrow color component in the visible spectrum, while being nonreflective, e.g. presenting a flat area of neutral color or being transmissive, outside that narrow wavelength band. The use of the infrared spectrum may further allow to extend the width of this band without discomfort, since no visual information is carried by the infrared light. This can be combined with adapting (s) light source(s) to the spectrum used for camera imaging (and corresponding to the reflective properties of the mirror), e.g. using infrared lighting. Obviously, these remarks do not apply to a part of the mirror assembly, earlier referred to as a third part thereof, if included in an embodiment, which is specifically adapted to assist in presenting such information provided via screen or projection to the subject.

The mirror assembly may be adapted for attaching to a piece of auxiliary equipment of the MRI scanner, such as an MRI coil assembly, e.g. a head-coil. The mirror assembly may alternatively integrated in such auxiliary equipment, e.g. form (a fixed) part of a coil assembly, e.g. a head-coil.

In a second aspect, the present invention relates to a head-coil attachment adapted for attaching to a radiofrequency transmitter/receiver head-coil assembly 12 for acquiring magnetic resonance signals from a subject's head region when examined by a magnetic resonance imaging system 1. The head-coil attachment comprises a mirror assembly 22 to reflect light from a target area on the body of the subject onto a camera of the magnetic resonance imaging system. The mirror assembly 22 comprises a curved reflective surface and/or a plurality of reflective surfaces oriented in different directions. The curved reflective surface and/or the plurality of reflective surfaces are adapted to reflect light from the target area into the camera by magnifying the reflected image and/or by projecting multiple copies of the same target area into different areas of an image acquired by the camera and/or to both reflect light from the target area into the camera as reflect light from at least one light source at a different position as the camera onto the target area. For example, the mirror assembly 22 may be a mirror assembly as discussed hereinabove in relation to the first aspect of the present invention.

In a further aspect, the present invention relates to an arm attachment (cf. the arm assembly discussed hereinabove), e.g. comprising one or more pliable structures and/or joints to provide some freedom in configuration of the orientation and/or position, adapted for attaching to a mount point on a (stationary part of a) gantry or magnet bore enclosure, or on a patient couch or other auxiliary equipment for use in the examination zone of a medical imaging system. Thus, the arm attachment is adapted for mounting in or on (i.e. attaching to; optionally removably attaching to) a medical imaging system (e.g. to a component thereof, e.g. to the patient couch) for examining a subject when positioned in an examination zone of the system. The arm attachment comprises a mirror assembly to reflect light from a target area on the body of the subject onto a camera of the medical imaging system. The mirror assembly comprises a curved reflective surface and/or a plurality of reflective surfaces oriented in different directions. The curved reflective surface and/or plurality of reflective surfaces are adapted to reflect light from the target area into the camera by magnifying the reflected image and/or by projecting multiple copies of the same target area into different areas of the image acquired by the camera and/or to both reflect light from the target area into the camera as reflect light from at least one light source at a different position as the camera onto the target area.

In a third aspect, the present invention relates to a radiofrequency transmitter/receiver coil assembly, e.g. a head-coil assembly 12, for acquiring magnetic resonance signals from a subject's body when examined by a magnetic resonance imaging system. The radiofrequency transmitter/receiver coil assembly comprising a mirror assembly 22 to reflect light from a target area on the body of the subject onto a camera of the magnetic resonance imaging system. The mirror assembly 22 comprises a curved reflective surface and/or a plurality of reflective surfaces oriented in different directions. The curved reflective surface and/or the plurality of reflective surfaces may be adapted to reflect light from the target area into the camera by magnifying the reflected image and/or by projecting multiple copies of the same target area into different areas of the image acquired by the camera and/or to both reflect light from the target area into the camera as reflect light from at least one light source at a different position as the camera onto the target area. For example, the mirror assembly 22 may be a mirror assembly as discussed hereinabove in relation to the first aspect of the present invention.

In a fourth aspect, the present invention relates to a method for monitoring a subject when undergoing a magnetic resonance imaging examination while positioned in an examination zone of a magnetic resonance imaging system, e.g. inside a magnet bore of the system.

FIG 10 shows an illustrative method 100 in accordance with embodiments of the present invention.

The method may comprise positioning the subject in the MRI system for examination.

The method may comprise imaging the subject using the MRI system, e.g. acquiring magnetic resonance signals from the subject in operation of the system.

The method comprises providing 101 a mirror assembly 22 in the examination zone to monitor the subject during the examination. For example, the mirror assembly may be integrated in or attached to a coil assembly, e.g. a head-coil.

The method comprises illuminating 102 a target area on the body of the subject, e.g. using one or more light sources, such as a light source outside or at an edge region of the examination zone, e.g. a bore light.

The target area may be illuminated by reflecting light from the light source onto the target area via a reflective surface of the mirror assembly.

The method comprises acquiring 103 at least one image by a camera 21 to observe the target area via a reflective surface of a first part of the mirror assembly . For example, the camera or cameras may be placed outside of at an edge region of the examination zone, e.g. in or on a flange of the magnet bore.

Acquiring the image may comprise observing the target area as magnified by a curved reflective surface of the mirror assembly and/or as replicated in different regions of the acquired image by reflections via a plurality of differently oriented reflective surfaces of the mirror assembly.

Other features, or details of the features described hereinabove, of a method in accordance with embodiments of the fourth aspect of the present invention shall be clear in view of the description provided hereinabove relating to a system in accordance with embodiments of the first aspect of the present invention.

**Claims**

1.  A medical imaging system (1) comprising:

    a camera (21) for monitoring a subject (14) when undergoing an examination while positioned in an examination zone (11) of the medical imaging system by acquiring at least one image, and
    a mirror assembly (22) arranged in the examination zone to reflect light from a target area on the body of the subject onto the camera,

wherein said mirror assembly (22) comprises a curved reflective surface and/or a plurality of reflective surfaces oriented in different directions, wherein said curved reflective surface and/or said plurality of reflective surfaces are adapted to reflect light from the target area into the camera by magnifying the reflected image and/or by projecting multiple copies of the same target area into different areas of the image acquired by the camera and/or to both reflect light from the target area into the camera as well as reflect light from at least one light source at a different position as the camera onto the target area.

2.  The medical imaging system of claim 1, wherein said mirror assembly (22) comprises a first mirror part (43), wherein the first mirror part comprises at least one reflective surface that is adapted to provide a view of the target area to the camera.

3.  The medical imaging system of claim 2, wherein said first mirror part (43) comprises a plurality of reflective surfaces (51, 52, 53), each adapted to reflect the same target area onto the camera, such that the target area is replicated via different optical paths in the acquired image.

4.  The medical imaging system of claim 1 or claim 2, wherein said first part (43) comprises a reflective surface forming a convergent mirror to provide a magnified view of the target area to the camera.

5.  The medical imaging system of claim 4, wherein said convergent mirror is a spherical or parabolic mirror, or has a spherical or parabolic profile in at least one direction.

6.  The medical imaging system of any of the claims 2 to 4, comprising at least one light source (29) for emitting a light beam into the examination zone, wherein said mirror assembly (22) comprises a second mirror part (44) comprising at least one reflective surface, which is oriented differently than the reflective surface of the first mirror part (43), to reflect the light from the light source onto the target area.

7.  The medical imaging system of claim 6, wherein said second mirror part (44) comprises a plurality of reflective surfaces with different orientations, such that light from a plurality of light sources is reflected onto the target area by corresponding reflective surfaces of the second mirror part.

8.  The medical imaging system of any of the previous claims, wherein said mirror assembly (22) comprises a third mirror part (45) for reflecting an image presented by a screen outside the examination zone (11) to the subject such that the subject can visually receive entertainment, instructions and/or information via said screen.

9. The medical imaging system of any of the previous claims, comprising a radiofrequency transmitter/receiver head-coil assembly (12) for acquiring magnetic resonance signals from the subject's head region and/or an arm assembly for attaching to a mount point on a gantry and/or on a magnet bore enclosure and/or on a patient couch and/or on other auxiliary equipment for use in the examination zone of the medical imaging system, wherein said mirror assembly (22) is adapted for attaching to, or is integrated in, the head coil assembly or the arm assembly.

10. The medical imaging system of any of the previous claims, wherein said camera (21) is integrated in, or mounted on, a flange end region of a magnet bore housing of the system.

11. The medical imaging system of any of the previous claims, comprising an image processor (27) adapted to process image information acquired by the camera to derive information about the subject.

12. The medical imaging system of claim 11, wherein said target area is a part of the forehead or a cheek of the subject or part thereof, and said image processor (27) is adapted to derive a photoplethysmography signal from images acquired by said camera (21).

13. A head-coil attachment adapted for attaching to a radiofrequency transmitter/receiver head-coil assembly (12) for acquiring magnetic resonance signals from a subject's head region when examined by a magnetic resonance imaging system, the head-coil attachment comprising a mirror assembly (22) to reflect light from a target area on the body of the subject onto a camera of the magnetic resonance imaging system,

wherein said mirror assembly (22) comprises a curved reflective surface and/or a plurality of reflective surfaces oriented in different directions, wherein said curved reflective surface and/or said plurality of reflective surfaces are adapted to reflect light from the target area into the camera by magnifying the reflected image and/or by projecting multiple copies of the same target area into different areas of the image acquired by the camera and/or to both reflect light from the target area into the camera as well as reflect light from at least one light source at a different position as the camera onto the target area.

14. A radiofrequency transmitter/receiver coil assembly (12) for acquiring magnetic resonance signals from a subject's body when examined by a magnetic resonance imaging system, the radiofrequency transmitter/receiver coil assembly comprising a mirror assembly (22) to reflect light from a target area on the body of the subject onto a camera of the magnetic resonance imaging system,

wherein said mirror assembly (22) comprises a curved reflective surface and/or a plurality of reflective surfaces oriented in different directions, wherein said curved reflective surface and/or said plurality of reflective surfaces are adapted to reflect light from the target area into the camera by magnifying the reflected image and/or by projecting multiple copies of the same target area into different areas of the image acquired by the camera and/or to both reflect light from the target area into the camera as well as reflect light from at least one light source at a different position as the camera onto the target area.

15. A method (100) for monitoring a subject (14) when undergoing a medical imaging examination while positioned in an examination zone (11) of a medical imaging system (1), the method comprising:

providing (101) a mirror assembly (22) in the examination zone,
illuminating (102) a target area on the body of the subject, and
acquiring (103) at least one image by a camera (21) to observe the target area via a reflective surface of a first part of the mirror assembly,
wherein said illuminating comprises reflecting light from a light source onto the target area via a reflective surface of a second part of the mirror assembly and/or
wherein said acquiring comprises observing the target area as magnified by a curved reflective surface of the first part of the mirror assembly and/or as replicated in different regions of the acquired image by reflections via a plurality of differently oriented reflective surfaces of the first part of the mirror assembly.

FIG 1

**FIG 2**

**FIG 3**

FIG 4

FIG 5

FIG 6

FIG 7

FIG 8

FIG 9

FIG 10

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y<br>A | US 2020/289075 A1 (ANDERSON KEVAN JAMES THOMPSON [CA] ET AL)<br>17 September 2020 (2020-09-17)<br>* paragraph [0147] – paragraph [0156];<br>figures 11C-11E * | 1,2,4-6,<br>8-11,<br>13-15<br>12<br>3,7 | INV.<br>G01R33/28<br>A61B6/03<br>A61N5/10 |
| Y<br><br><br><br><br><br><br><br><br><br><br>A | SPICHER NICOLAI ET AL: "Heart rate monitoring in ultra-high-field MRI using frequency information obtained from video signals of the human skin compared to electrocardiography and pulse oximetry", CURRENT DIRECTIONS IN BIOMEDICAL ENGINEERING,<br>vol. 1, no. 1,<br>12 September 2015 (2015-09-12), pages 69-72, XP055865602,<br>DOI: 10.1515/cdbme-2015-0018<br>* page 1 – page 2 * | 12<br><br><br><br><br><br><br><br><br><br>1-11,<br>13-15 | ADD.<br>A61B6/00<br>A61B5/024<br>A61B5/08 |

TECHNICAL FIELDS
SEARCHED        (IPC)

G01R
A61B
A61N

The present search report has been drawn up for all claims

| Place of search | Date of completion of the search | Examiner |
|---|---|---|
| Munich | 23 December 2021 | Raguin, Guy |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
 
& : member of the same patent family, corresponding
document

EPO FORM 1503 03.82 (P04C01)

## EP 4 124 875 A1

### ANNEX TO THE EUROPEAN SEARCH REPORT
### ON EUROPEAN PATENT APPLICATION NO.

EP 21 18 7585

This annex lists the patent family members relating to the patent documents cited in the above-mentioned European search report.
The members are as contained in the European Patent Office EDP file on
The European Patent Office is in no way liable for these particulars which are merely given for the purpose of information.

23-12-2021

| Patent document cited in search report | Publication date | Patent family member(s) | Publication date |
|---|---|---|---|
| US 2020289075 A1 | 17-09-2020 | CN 111386496 A | 07-07-2020 |
| | | EP 3692416 A1 | 12-08-2020 |
| | | JP 2020535931 A | 10-12-2020 |
| | | US 2020289075 A1 | 17-09-2020 |
| | | WO 2019068185 A1 | 11-04-2019 |

EPO FORM P0459

For more details about this annex : see Official Journal of the European Patent Office, No. 12/82

**EP 4 124 875 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0928689 B